(19) Europäisches Patentamt
European Patent Office
Office européen des brevets

(11) **EP 2 609 854 B1**

(12) **EUROPÄISCHE PATENTSCHRIFT**

(45) Veröffentlichungstag und Bekanntmachung des Hinweises auf die Patenterteilung:
**14.07.2021   Patentblatt 2021/28**

(51) Int Cl.:
*A61B 5/00* *(2006.01)*       *A61B 5/024* *(2006.01)*
*A61B 5/026* *(2006.01)*       *A61B 5/1455* *(2006.01)*

(21) Anmeldenummer: **12199633.4**

(22) Anmeldetag: **28.12.2012**

(54) **Bewegungskorreliertes Verfahren und opto-elektronische Vorrichtung zur nichtinvasiven Bestimmung der dermalvenösen Sauerstoffversorgung peripherer Beingebiete**

Movement correlated method and optoelectronic device for non-invasive determination of the dermal venous oxygen supply to peripheral areas of the leg

Procédé corrélé au mouvement et dispositif optoélectronique destiné à la détermination non invasive de l'alimentation en oxygène des veines du derme de zones périphériques des jambes

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.12.2011   DE 102011122700**

(43) Veröffentlichungstag der Anmeldung:
**03.07.2013   Patentblatt 2013/27**

(73) Patentinhaber: **Blazek, Vladimir**
**52074 Aachen (DE)**

(72) Erfinder:
• **Blazek, Vladimir**
  **52074 Aachen (DE)**

• **Blazek, Claudia R.**
  **8001 Zürich (CH)**

(56) Entgegenhaltungen:
**DE-A1- 4 238 641       US-B1- 6 334 065**

• **ASADA H H ET AL: "Mobile monitoring with wearable photoplethysmographic biosensors", IEEE ENGINEERING IN MEDICINE AND BIOLOGY MAGAZINE, IEEE SERVICE CENTER, PISACATAWAY, NJ, US, Bd. 22, Nr. 3, 1. Mai 2003 (2003-05-01), Seiten 28-40, XP011098587, ISSN: 0739-5175, DOI: 10.1109/MEMB.2003.1213624**

• **None**

## Beschreibung

Die Erfindung bezieht sich auf die nichtinvasive Erfassung der arteriellen und venösen Hämodynamik (Blutveränderung als Funktion der Zeit) in bewegten und ruhenden menschlichen Extremitäten.

Eine gattungsgemäße Vorrichtung ist beispielsweise aus der DE 3100610 bekannt. Diese Vorrichtung weist einen Sensor auf, der wenigstens eine Lichtquelle und einen Lichtdetektor beinhaltet. Auf diese Druckschrift wird im Übrigen zur Erläuterung aller hier nicht näher beschriebenen Begriffe ausdrücklich verwiesen. Außerdem wird Bezug genommen auf bekannte photoplethysmographische Messsysteme, welche durch Auswertung peripherer arterieller Blutvolumenpulsationen nach dem pulsoximetrischen Verfahren die arterielle Sauerstoffsättigung ermitteln. In der US 6,334,065 B1 wird ein Pulsoximeter beschrieben, mit dem eine Bestimmung der venösen Sauerstoffsättigung erfolgt. Dabei wird ein externer Pulsgeber verwendet, um das Blutvolumen zu modulieren. Alternativ kann der Atemrhythmus als Pulsgeber verwendet werden.

Bei der funktionellen Beurteilung der peripheren venösen Hämodynamik unter Muskelarbeit hat neben der Ultraschall-Doppler-Sonographie vor allem auch die Photoplethysmographie (kurz PPG) in den letzten drei Dekaden eine große Ausbreitung gefunden. Auf Grund ihrer einfachen und schnellen Handhabung lässt sich mit der PPG neben der Früherkennung von Beinvenenerkrankungen auch die hämodynamische Auswirkung besonderer Belastungssituationen, wie z.B. Schwangerschaft und Wochenbett, quantifizieren und die Erhebung epidemiologischer Venenparameter durchführen. Die PPG stellt somit eine Methode dar, die sowohl in der täglichen Routine als auch in der klinischen Forschung breite Anwendung bietet.

Der methodischen Basisgrundlage der PPG liegt die Tatsache zu Grunde, dass selektives Licht im sichtbaren oder nahen Infrarotbereich von Hämoglobin bzw. von Blut wesentlich stärker als von Gewebe absorbiert wird. Da z.B. eine Gefäßerweiterung mit einer Zunahme des Blutvolumens verbunden ist, vergrößert sich zwangsläufig auch das Absorptionsvolumen. Sendet man das energiearme selektive Licht in das Gewebe hinein, so registriert ein Detektor im PPG-Sensor (auch Optrode genannt) mit Zunahme des Blutvolumens im Messvolumen unter dem Sensor weniger Licht. Das PPG-Signal spiegelt somit Blutvolumenschwankungen in kutanen und subkutanen Gefäßnetzen wieder und setzt sich in erster Näherung aus einem sehr großen durchblutungsunabhängigen gleich bleibenden Anteil (Streuung im Gewebe), einem quasistatischen Venensignal und einem sehr geringen, periodischen Arteriensignal zusammen.

Unter physiologischen Bedingungen strömt das Venenblut von "körperfern zu körpernah" und von der Hautoberfläche in Körpertiefe. Der postkapillare Restdruck einerseits und die Ansaugung durch respiratorische Druckschwankungen im Brust- und Bauchraum bzw. durch die Herzaktion andererseits gewährleisten eine derartige Strömung im Liegen.

Beim Sitzen, Stehen und Gehen muss das Blut in den Beinvenen aber "bergauf" fließen. Dieses logistische Transportproblem wird bei den Venengesunden dadurch spielend bewältigt, dass zu den vorgenannten Fördermechanismen der normalen Blutströmung "herzwärts" zusätzlich die sogenannte Muskel-Gelenk-Pumpe aktiviert wird. Die Funktion der einzelnen Muskelgruppen des Beines und die Wirkung der Gelenke sind untereinander so fein abgestimmt, dass die notwendige Rückflussenergie gewonnen werden kann. Die in den Venen vorhandenen Ventile - die Venenklappen - verhindern dabei im Normalfall in der Orthostase die Rückströmung des Blutes.

Das Hauptanwendungsgebiet der venösen Photoplethysmographie liegt deshalb in der funktionellen Überprüfung der globalen Bluttransporteigenschaften des Beinvenensystems unter Muskelarbeit. Bei dem standardisierten Muskelpumpen-Test führt der Patient im Sitzen mehrere Dorsalextensionen im Sprunggelenk bei am Boden abgestützter Ferse ("Fußwippübungen") durch. Bei der hier beschriebenen Messanordnung werden bevorzugt 15 aktive Beinübungen in 30 Sekunden vom Patienten durchgeführt. Aber auch "passive" Beinübungen, wie etwa eine periodische externe Kontraktion des Unterschenkels oder periodischer Lagewechsel der Extremität ist möglich, vor allem beim Patienten ohne Beweglichkeit im Sprunggelenk.

Der PPG-Sensor wird vorzugsweise flach ausgeführt und etwa 10 cm oberhalb des Innenknöchels befestigt. Ebenso kann der flexible Sensor z.B. auch im Bereich der Fußsohle oder an den Zehen fixiert werden. Wegen seiner flachen Bauweise kann die Untersuchung auch mit angelegten Kompressionsstrümpfen vorgenommen werden.

Bei Venengesunden kann das abgepumpte Blut infolge der Venenklappen nicht einfach wieder zurückfallen. Es kommt deshalb zu einer deutlichen Venenentleerung in den Füßen und Unterschenkeln, die über eine Verkleinerung des Gefäßdurchmessers zu erhöhter Reflektivität der Haut und damit zum Anstieg des gesamten PPG-Signals führt. In der Ruhephase nach Ende der Übung füllen sich die Beinvenen im Normalfall durch den arteriellen Einstrom wieder auf, so dass die PPG-Kurve relativ langsam in die Nähe der Basislinie absinkt. Bei Venenkranken führt der pathologische Reflux des Blutes in den Beinvenen (Venenklappen undicht, defekt oder nicht vorhanden) zu einer deutlich schnelleren Auffüllung und damit zu einer Verkürzung der Wiederauffüllphase.

Für die Diagnosestellung können u. a folgende funktionelle Bewertungsparameter der PPG-Kurve entnommen werden:

- venöse Auffüllzeit To in Sekunden und

- venöse Pumpleistung Vo in Prozent des optischen Signals (Reflexionsänderung der Haut, bezogen auf die Ruhesignalamplitude (DC-Wert) vor Beginn der Übung).

In Anlehnung an bisherige Verfahren gilt die venöse Auffüllzeit (Zeitintervall vom Kurvenmaximum nach Ende der Bewegung bis zum Wiedererreichen einer konstanten Hautdurchblutung) als zurzeit wichtigster venöser Bewertungsparameter der PPG-Kurve. Je nach Verkürzung der Auffüllzeit, z.B. in Folge pathologischer Blutrefluxe in insuffizienten Beinvenen, wurde für die sitzende Beinübung folgende hämodynamische Schweregradeinteilung eingeführt:

| | |
|---|---|
| Normale Hämodynamik (venengesund): | To $\geq$ 25 Sekunden |
| Grad I (leichte hämodynamische Störung): | $20 \leq$ To $<$ 25 Sekunden |
| Grad II (mittelschwere hämodynamische Störung): | $10 \leq$ To $<$ 20 Sekunden |
| Grad III (schwere hämodynamische Störung): | To $<$ 10 Sekunden |

Da es bei den peripheren Gefäßerkrankungen oft um Erkrankungen gemischter Genese handelt, also venös und arteriell, erscheint es sowohl medizinisch-diagnostisch als auch bio-physikalisch sehr sinnvoll, neben dem o.e. Zeitparameter To auch die arteriellen **und** venösen Sauerstoffsättigungswerte in einem Untersuchungsgang zu bestimmen.

Wie allgemein bekannt, gibt die photoplethysmographisch/pulsoximetrisch ermittelte arterielle Sauerstoffsättigung ($S_pO_2$) an, wie viel Prozent des gesamten Hämoglobins im Blut mit Sauerstoff beladen ist. Dieser Sättigungswert ist ein wichtiger diagnostischer Parameter zur kombinierten Beurteilung der Atemfunktion und des peripheren dermalen Durchblutungsstatus bei Neonaten, Kindern und Erwachsenen gleicher maßen. Das Prinzip der Pulsoximetrie (s. z.B. http://de.wikipedia.org/wiki/Pulsoximetrie) wird hier als bekannt vorausgesetzt. Neben der Bestimmung der $S_pO_2$ wird in der klinischen Praxis auch die Sauerstoffsättigung im venösen Blut verschiedener Stromgebiete untersucht, allerdings bis dato nur invasiv. Aus der Differenz der arteriellen und venösen Sättigung lässt sich unter Berücksichtigung der Durchblutung die Sauerstoffaufnahme des Gewebes abschätzen. Setzt man hingegen eine konstante Sauerstoffaufnahme voraus, weist eine Zunahme der Differenz auf eine Abnahme der Durchblutung hin (http://de.wikipedia.org/wiki/Sauerstoffsättigung).

Es ist u.a. eine Aufgabe der Erfindung, eine Vorrichtung zur nichtinvasiven Bestimmung der venösen Sauerstoffsättigung derart weiterzubilden, dass diese bisher nicht messbaren Parameter aus der dermalen bewegungs- und herzsynchronen Blutperfusionsänderung in menschlichen Extremitäten unter aktiver oder passiver Muskelarbeit erfasst, analysiert und diese dem Arzt angezeigt werden können.

Gemäß einem ersten Aspekt der Erfindung wird eine Vorrichtung zur nichtinvasiven Bestimmung der venösen Sauerstoffsättigung offenbart, mit mindestens einer, vorzugsweise zwei Strahlungsquellen zur Erzeugung von Strahlung mit mindestens zwei Messwellenlängen, wobei die Strahlung ein Messareal einer Extremität beaufschlagt, mit mindestens einem Detektor, der die reflektierte und/oder zurückgestreute und/oder transmittierte Strahlung empfängt, und mit einer mit dem Detektor verbundenen Auswerteelektronik zur Analyse mindestens eines Detektorausgangssignals (also einem Ausgangssignal des Detektors), dadurch gekennzeichnet, dass die Auswerteelektronik zumindest eine ersten Filtereinheit aufweist und eingerichtet ist, das mindestens eine Detektorausgangssignal derart zu analysieren, dass ein DC- und ein AC-Anteil der venösen, bewegungskorrelierten Blutvolumenänderung in der Extremität bei jeder der verwendeten Messwellenlängen selektiv ermittelbar ist, was wiederum die Bestimmung der venösen Sauerstoffsättigung ermöglicht, wobei die bewegungskorrelierte Blutvolumenänderung durch passive und/oder aktive Bewegung der Extremität verursacht ist, wobei die erste Filtereinheit an eine Frequenz des venösen Signals synchron mit der oder gesteuert durch die Bewegung der Extremität angepasst ist und durch frequenzselektive Bewertung den DC- und den AC-Anteil ermittelt.

Gemäß einem weiteren Aspekt der Erfindung wird ein Verfahren zur nichtinvasiven Bestimmung der venösen Sauerstoffsättigung offenbart, umfassend die folgenden Schritte:

- Beaufschlagen eines Messareals einer Extremität, in welcher durch passives und/oder aktives Bewegen eine bewegungskorrelierte Blutvolumenänderung hervorgerufen wird, mit Strahlung mit mindestens zwei Messwellenlängen,
- Empfangen der reflektierten und/oder zurückgestreuten und/oder transmittierten Strahlung mit mindestens einem Detektor und
- Analysieren mindestens eines Detektorsignals (also einem Ausgangssignal des Detektors), sodass ein DC- und ein AC-Anteil der venösen, bewegungskorrelierten Blutvolumenänderung in der Extremität bei jeder der verwendeten Messwellenlängen selektiv ermittelt wird, und Bestimmen der venösen Sauerstoffsättigung basierend auf dem ermittelten DC- und AC-Anteil.

Das passive und/oder aktive Bewegen der Extremität kann beispielsweise ebenfalls ein Schritt des Verfahrens sein.

Gemäß einem weiteren Aspekt wird die Verwendung einer Vorrichtung oder eines Systems zur nichtinvasiven Bestimmung der venösen Sauerstoffsättigung einer Extremität, in der durch passive und/oder aktive Bewegung eine bewegungskorrelierte Blutvolumenänderung verursacht wird, offenbart, wobei die Vorrichtung oder das System folgendes umfasst:

- mindestens eine, vorzugsweise zwei Strahlungsquellen zur Erzeugung von Strahlung mit mindestens zwei Messwellenlängen, wobei die Strahlung ein Messareal der Extremität beaufschlagt,
- mindestens ein Detektor, der die reflektierte und/oder zurückgestreute und/oder transmittierte Strahlung empfängt, und
- eine mit dem Detektor verbundene Auswerteelektronik zur Analyse mindestens eines Detektorausgangssignals (also einem Ausgangssignal des Detektors), wobei die Auswerteelektronik eingerichtet ist, das mindestens eine Detektorausgangssignal derart zu analysieren, dass ein DC- und ein AC-Anteil der venösen, bewegungskorrelierten Blutvolumenänderung in der Extremität bei jeder der verwendeten Messwellenlängen selektiv ermittelbar ist, was wiederum die Bestimmung der venösen Sauerstoffsättigung ermöglicht.

Im Folgenden werden beispielhafte Ausführungsformen der Erfindung beschrieben, die gleichermaßen als für die verschiedenen Aspekte der Erfindung offenbart verstanden werden sollen.

In einer beispielhaften Ausführungsform wird das Bewegen der Extremität nicht durch die Vorrichtung bzw. durch das System durchgeführt, gesteuert oder veranlasst. Beispielsweise erfolgt das Bewegen der Extremität vollkommen unabhängig von der Vorrichtung bzw. von dem System. Beispielsweise werden insbesondere keine Hilfsmittel zum Erzeugen der Bewegung verwendet. Insbesondere arbeiten die Vorrichtung bzw. das System nicht mit Hilfsmitteln zur künstlichen Erzeugung einer Blutbewegung (beispielsweise Staumanschetten od. ähnlichem).

In einer beispielhaften Ausführungsform der Erfindung wird das Bewegen der Extremität durch einen Menschen vorgenommen, insbesondere unmittelbar durch einen Menschen. Bei dem Mensch kann es sich beispielsweise um den Besitzer der Extremität handeln, der diese selbst bewegt, oder um einen Menschen, der die Extremität eines anderen Menschen bewegt. Bei dem Bewegen der Extremität kann es sich beispielsweise um eine standardisierte Bewegung handeln.

In einer beispielhaften Ausführungsform der Erfindung beruht die Bestimmung der Sauerstoffsättigung auf reflektierter und/oder zurückgestreuter Strahlung. Beispielsweise sind die ein oder mehreren Strahlungsquellen und der Detektor im Wesentlichen auf einer Seite des Messareals angebracht, und beispielsweise nicht auf gegenüberliegenden Seiten des Messareals. Es erfolgt dann beispielsweise im Wesentlichen eine Reflexionsmessung und beispielsweise keine Transmissionsmessung. Eine Reflexionsmessung kann beispielsweise lediglich dann sinnvolle Ergebnisse liefern, wenn sie an einer bewegten Extremität vorgenommen wird.

In einer beispielhaften Ausführungsform der Erfindung beruht die Bestimmung der Sauerstoffsättigung nicht auf transmittierter Strahlung.

In einer beispielhaften Ausführungsform der Erfindung sind die Strahlungsquelle(n) und der Detektor in einem Sensor enthalten.

In einer beispielhaften Ausführungsform der Erfindung beträgt eine erste der beiden Messwellenlängen 940 nm und eine zweite der beiden Messwellenlängen beträgt 660 nm.

In einer beispielhaften Ausführungsform der Erfindung ist die Auswerteeinheit eine Steuer- und Auswerteeinheit.

In einer beispielhaften Ausführungsform der Erfindung ist die Extremität ein Bein oder ein Teil davon. Im Gegensatz zu einer Messung an einem Finger ergibt sich hier der Vorteil, dass bei einer Beinübung die Muskelpumpe im wesentlichen nur das venöse Blut im Bein (Fuss- und Unterschenkelbereich) nach oben bewegt und das venöse Blutvolumen dort vielfach höher ist als das arterielle. Die Messergebnisse erlauben damit eine genauere Messung des venösen Sättigungswerts als bei einer Messung an einem Finger, wo das arterielle Blutvolumen und das venöse Blutvolumen annähernd gleich sind und daher das Messergebnis des venösen Sättigungswerts viel stärker durch den arteriellen Sättigungswert verfälscht ist.

In einer beispielhaften Ausführungsform der Erfindung sind zumindest die Strahlungsquelle(n) und der Detektor in einem Kleidungsstück, insbesondere einem Fußkleidungsstück, beispielsweise einem Strumpf, integriert.

In einer beispielhaften Ausführungsform der Erfindung umfasst das Analysieren des mindestens einen Detektorausgangssignals Digitalisieren und Filtern.

In einer beispielhaften Ausführungsform der Erfindung ist die Auswerteelektronik eingerichtet, die venöse Sauerstoffsättigung im transilluminierten Gewebevolumen unter dem Sensor zu bestimmen.

In einer beispielhaften Ausführungsform der Erfindung ist die Vorrichtung eine opto-elektronische Vorrichtung.

In einer beispielhaften Ausführungsform der Erfindung ist die Vorrichtung eine Vorrichtung zur Bestimmung der dermalvenösen Sauerstoffsättigung, die zur Bestimmung der Sauerstoffsättigung von oberen und/oder unteren Extremitäten, insbesondere peripherer Beingebiete, geeignet und/oder eingerichtet ist.

In einer beispielhaften Ausführungsform der Erfindung umfasst das Messareal darunterliegende Schichten.

In einer beispielhaften Ausführungsform der Erfindung ist der Sensor in flacher und/oder biegsamer Bauweise ausgeführt, so dass insbesondere seine Anpassung an das gewählte Gewebearealprofil gewährleistet ist und somit die Detektion der Strahlung sowohl im Reflexions- als auch Transmissionsmodus stattfinden kann

In einer beispielhaften Ausführungsform der Erfindung ist die Auswerteeinheit detektornah (also sensornah, wenn der Detektor von einem Sensor umfasst ist) angeordnet und es finden keine spektralen Manipulationen an dem mindestens einen gemessenen (elektrischen) Detektorausgangssignal, insbesondere dem Perfusionssignal, statt, sodass insbesondere hämodynamische Informationsverluste reduziert, insbesondere ausgeschlossen, werden.

In einer beispielhaften Ausführungsform beruht die Ermittlung des DC- und AC-Anteils der venösen, bewegungskorrelierten Blutvolumenänderung zumindest teilweise (oder vollständig) auf der Analyse eines (beispielsweise ausschließlich) während dem passiven und/oder aktiven Bewegen der Extremität erfassten Detektorsignals (bzw. der von dem Detektor während dem passiven und/oder aktiven Bewegen der Extremität empfangenen Strahlung).

In einer beispielhaften Ausführungsform wird das mindestens eine Detektorausgangssignal zwei unterschiedlichen digitalen Filtereinheiten zugeführt (die aber auch in einer Einheit zusammengefasst sein können), wo zumindest durch frequenzselektive Bewertung die DC- und AC-Anteile der arteriellen und venösen (Perfusions-)Signale (beispielsweise der arteriellen und venösen Perfusionssignale, die beispielsweise repräsentativ für die arterielle und venöse Blutvolumenänderung sind) errechnet werden, vorzugsweise für alle verwendeten Messwellenlängen getrennt. Beispielsweise kann eine erste der beiden digitalen Filtereinheiten an eine Frequenz des venösen Signals (z.B. 0,5 Hz, beispielsweise synchron mit der Beinbewegung oder gesteuert/getriggert durch die Beinbewegung) und eine zweite der beiden digitalen Filtereinheiten an eine Frequenz (z.B. 1 Hz, beispielsweise synchron mit dem Herzschlag oder gesteuert/getriggert durch den Herzschlag) des arteriellen Signals angepasst sein. Beispielsweise können die digitalen Filtereinheiten auch eine zeitselektive Zuordnung vornehmen. Beispielsweise kann während mindestens einer Ruhephase, die beispielsweise einer Phase, in der die passive und/oder aktive Bewegung der Extremität stattfindet, vorangeht oder folgt, das Detektorsignal analysiert werden, um einen DC-Anteil und einen AC-Anteil der arteriellen Blutvolumenänderung (bzw. des arteriellen Perfusionssignals) zu bestimmen und während der Phase, in der die passive und/oder aktive Bewegung der Extremität erfolgt, das Detektorsignal analysiert werden, um den DC-Anteil und den AC-Anteil der venösen bewegungskorrelierten Blutvolumenänderung (bzw. des venösen Perfusionssignals) zu bestimmen.

In einer beispielhaften Ausführungsform wird das mindestens eine Detektorausgangssignal einer digitalen Filtereinheit zugeführt, wo zumindest durch frequenzselektive Bewertung die DC- und AC-Anteile des venösen (Perfusions-)Signals errechnet werden, vorzugsweise für alle verwendeten Messwellenlängen getrennt. Beispielsweise kann die digitale Filtereinheit (nachfolgend als erste digitale Filtereinheit bezeichnet) an eine Frequenz des venösen Signals (z.B. 0,5 Hz) angepasst sein. Eine weitere digitale Filtereinheit (nachfolgend als zweite digitale Filtereinheit bezeichnet, die aber mit der ersten digitalen Filtereinheit auch eine Einheit bilden kann) kann beispielsweise der Bestimmung eines DC-Anteils und eines AC-Anteils des arteriellen (Perfusions-)Signals dienen und beispielsweise an eine Frequenz des arteriellen (Perfusions-)Signals angepasst sein. Beispielsweise kann während mindestens einer Ruhephase, die beispielsweise einer Phase, in der die passive und/oder aktive Bewegung der Extremität stattfindet, vorangeht oder folgt, das Detektorsignal mit der zweiten digitalen Filtereinheit analysiert werden, um einen DC-Anteil und einen AC-Anteil der arteriellen Blutvolumenänderung (bzw. des arteriellen Perfusionssignals) zu bestimmen und während der Phase, in der die passive und/oder aktive Bewegung der Extremität erfolgt, das Detektorsignal mit der ersten digitalen Filtereinheit analysiert werden, um den DC-Anteil und den AC-Anteil der venösen bewegungskorrelierten Blutvolumenänderung (bzw. des venösen Perfusionssignals) zu bestimmen. DC- und AC-Anteil der arteriellen Blutvolumenänderung können beispielsweise aber auch in der Ruhephase und in der Bewegungsphase bestimmt werden.

In einer beispielhaften Ausführungsform der Erfindung sind eine oder mehrere weitere Auswerteeinheiten vorgesehen, um aus den venösen und arteriellen DC- und AC-Wertepaaren sowohl die arterielle als auch die venöse Sauerstoffsättigung zu bestimmen.

In einer beispielhaften Ausführungsform der Erfindung wird aus den Werten der arteriellen und venösen Sauerstoffsättigung transkutan die Sauerstoffsättigungsdifferenz zwischen dem arteriellen und dem venösen Gefäßbett bestimmt.

In einer beispielhaften Ausführungsform der Erfindung ist die Bewegung der Extremität eine aktive Beinübung durch den Patienten und/oder eine externe Beinübung durch den Untersucher (manuelle Blutvolumendrainage). Die Bewegung der Extremität kann beispielsweise unter aktiver oder passiver Muskelarbeit erfolgen. Die Bewegung der Extremität kann beispielsweise die Bewegung eines Teils der Extremität relativ zu einem weiteren Teil der Extremität beinhalten, wobei die beiden Teile der Extremitäten beispielsweise durch ein Gelenk verbunden sind. Es kann sich also beispielsweise um eine Bewegung eines Fußes relativ zu einem Unterschenkeln oder einer Hand relativ zu einem Unterarm oder eines Fingers relativ zu einer Hand oder eines Fingergliedes relativ zu einem anderen Fingerglied handeln. Die Bewegung der Extremität kann beispielsweise Dorsalextensionen im Sprunggelenk abgestützter Ferse ("Fußwippübungen") umfassen.

In einer beispielhaften Ausführungsform der Erfindung ist die Bewegung der Extremität eine passive Beinübung durch wiederholte Hoch- und Tieflagerung der Extremität.

In einer beispielhaften Ausführungsform der Erfindung ist die Bewegung der Extremität eine Bewegung der oberen Extremität, vorzugsweise einer Fingerspitze oder eines Daumenballens.

In einer beispielhaften Ausführungsform der Erfindung ist die Innenseite der Sensoroberfläche (zum Gewebe hin) aus weichem und/oder rutschfestem Material ausgebildet.

In einer beispielhaften Ausführungsform der Erfindung ist die Außenseite der Sensoroberfläche (weg vom Gewebe) aus lichtundurchlässigem Material ausgebildet und zwischen den beiden äußeren flexiblen Sensorschichten sind ebenfalls flexible leitende und geerdete Abschirmungsfolien eingearbeitet.

In einer beispielhaften Ausführungsform der Erfindung ist die Frequenz der Bewegung der Extremität geringer als 1 Hz, vorzugsweise etwa 0,5 Hz.

In einer beispielhaften Ausführungsform der Erfindung wird die Frequenz der Bewegung der Extremität durch die Vorrichtung oder das System aus dem mindestens einen Detektorausgangssignal ermittelt. Dazu wird beispielsweise das Detektorausgangssignal während der Bewegungsphase auf Minima und Maxima analysiert und aus deren Abstand die Bewegungsfrequenz bestimmt.

In einer beispielhaften Ausführungsform der Erfindung wird neben der Bestimmung der venösen Sauerstoffsättigung (und beispielsweise auch der arteriellen Sauerstoffsättigung) auch (beispielsweise gleichzeitig) die venöse Auffüllzeit bestimmt (beispielsweise basierend auf den gleichen Messwerten oder Detektorsignalen, die beispielsweise zumindest teilweise während einer standardisierten Beinübung, beispielsweise dem sog. Muskelpumpen-Test, erfasst werden).

Die vorgenannten beispielhaften Ausführungsformen und deren einzelne Merkmale sollen auch in allen möglichen Kombinationen miteinander offenbart verstanden werden.

Die Erfindung wird nachstehend ohne Beschränkung des allgemeinen Erfindungsgedankens anhand von Ausführungsbeispielen unter Bezugnahme auf die Zeichnungen exemplarisch beschrieben, auf die im Übrigen bezüglich der Offenbarung aller im Text nicht näher erläuterten erfindungsgemäßen Einzelheiten ausdrücklich verwiesen wird. Es zeigen:

Fig. 1    die bevorzugte Position der optisch-elektronischen Sensoreinheit auf dem Unterschenkel respektive Fuß des sitzenden Probanden und das Blockschaltbild eines Ausführungsbeispiels der Vorrichtung mit den wichtigsten Systemkomponenten,

Fig. 2    typische Signalbeispiele (arterielle Photoplethysmogramme bei ruhendem Bein) der erfindungsgemäßen Vorrichtung, gleichzeitig bei zwei verschiedenen Messwellenlängen gemessen,

Fig. 3    spektrale Darstellung des molaren Extinktionsverhaltens vom oxigenierten und reduziertem Hämoglobin im Wellenlängenbereich vom 600 bis 1000 nm,

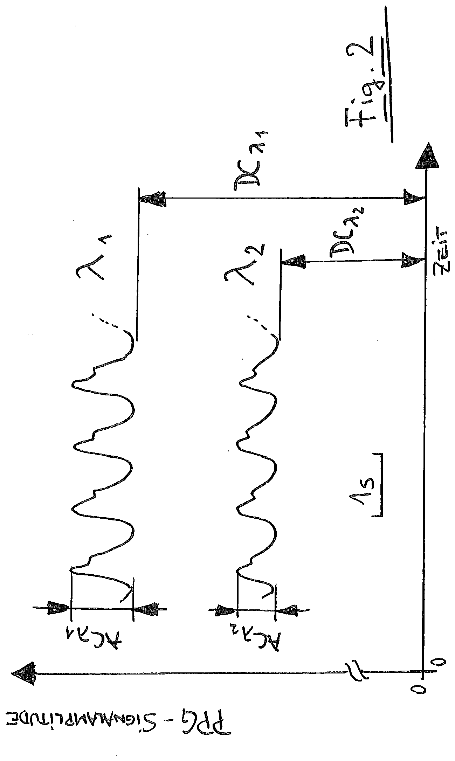

Fig. 2    typische ungefilterte Perfusionssignalbeispiele (mit arteriellen und venösen Signalanteilen) der erfindungsgemäßen Vorrichtung, gleichzeitig bei zwei verschiedenen Messwellenlängen gemessen in der initialen Ruhephase, gefolgt von einer 30 Sekunden langen Beinbewegungsphase und anschließender Ruhephase,

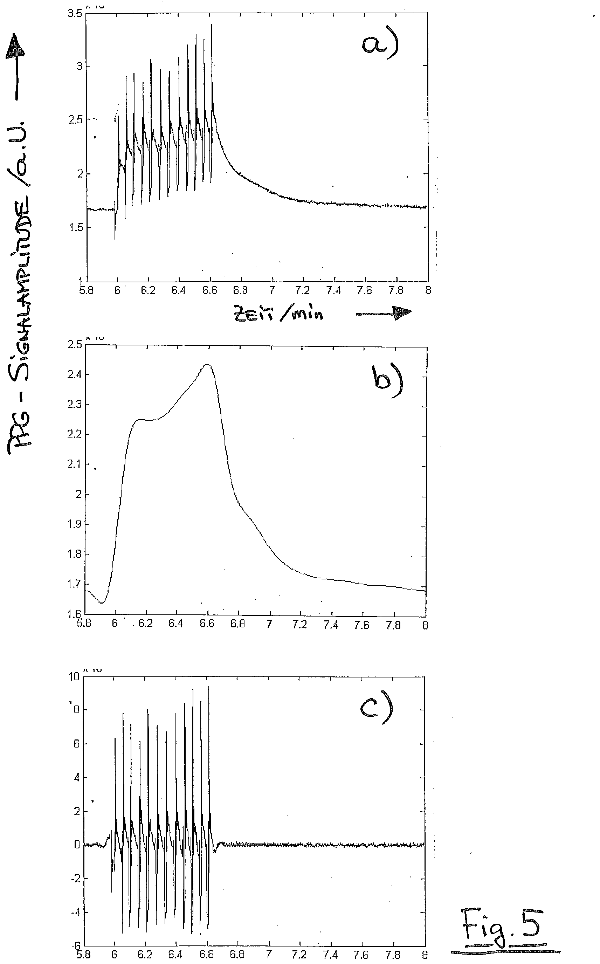

Fig. 5    typische venöse Signalbeispiele der erfindungsgemäßen Vorrichtung, gemessen bei einer der beiden Messwellenlängen. Neben dem photoplethysmographischen Gesamtsignal (a) sind der durch digitale Filterung daraus ermittelte venöse DC-Anteil (b) und der venöse AC-Anteil (c) dargestellt,

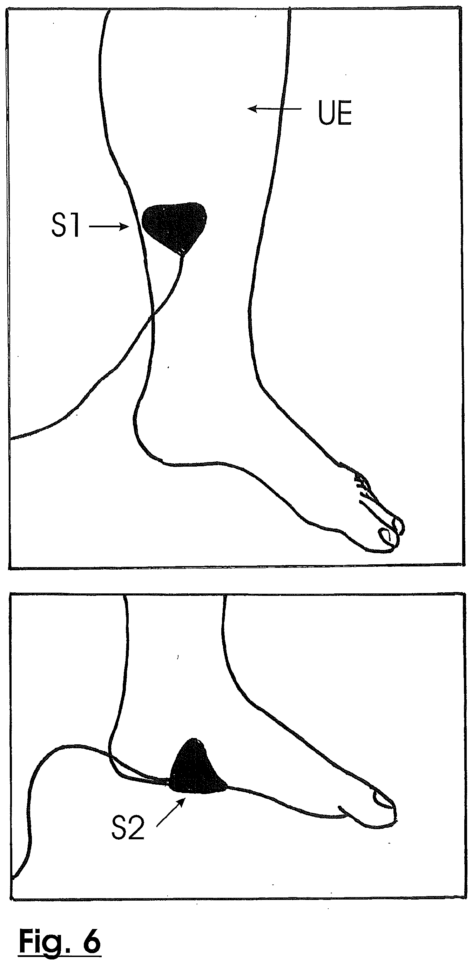

Fig. 6    die bevorzugte Sensorform und Applikationspositionen am Patientenbein.

Ausführungsbeispiele der vorliegenden Erfindung betreffen ein bewegungskorreliertes Verfahren und eine opto-elektronische Vorrichtung zur nichtinvasiven (transkutanen) Bestimmung der dermalen venösen Sauerstoffsättigung im Bereich der menschlichen Extremitäten, bevorzugt im Bereich der Beine. Die Vorrichtung umfasst (beispielsweise in ihrer Minimalausstattung) folgendes (und das Verfahren umfasst entsprechende Schritte):

- einen flachen, biegsamen Sensor, welcher je nach gewähltem Gewebeareal im Reflexions- oder Transmissionsmodus arbeiten kann, mit mindestens einem Photodetektor und zwei selektiven Lichtquellen, deren Licht aufeinander abgestimmt ist, das Hautareal transilluminiert,
- einen sensornahen Steuer- und Auswerteeinheit, an der die elektrischen Ausgangssignale des Sensors anliegen und hier hochaufgelöst digitalisiert werden, so dass die überlagerten arteriellen und venösen Perfusionssignale am Ausgang ohne hämodynamische Informationsverluste am Ausgang der Einheit zur weiteren Verarbeitung zur Verfügung stehen,

- eine digitale Filtereinheit, welche während der Beinbewegung die venösen (und optional auch die arteriellen) DC- und AC-Signalanteile für beide verwendeten Messwellenlängen getrennt ermittelt, und

- weitere rechnerunterstützte Auswerteeinheiten, welche aus den venösen und arteriellen DC- und AC-Signalanteilen neben der bekannten arteriellen Sauerstoffsättigung ($S_pO_2$) auch die venöse Sauerstoffsättigung ($S_bO_2$) im Hautareal unter dem Sensor errechnet.

Zusätzlich kann auch noch eine weitere digitale Filtereinheit vorhanden sein, welche bei ruhendem Patienten die arteriellen DC- und AC-Signalanteile für beide verwendeten Messwellenlängen getrennt ermittelt.

Durch die abgestimmte, erfindungsgemäße Zusammenwirkung ihrer einzelnen Merkmale zeichnet sich die beschriebene Vorrichtung im Vergleich mit den photoplethysmographischen Vorrichtungen nach dem Stande der Technik beispielsweise dadurch aus, dass neben dem gefäßdiagnostisch relevanten hämodynamischen Bewertungsparameter "venöse Auffüllzeit To" nach standardisierter Beinübung (sogenannter Muskelpumpen-Test) gleichzeitig auch die venösen und arteriellen Sauerstoffsättigungswerte ermittelt werden können.

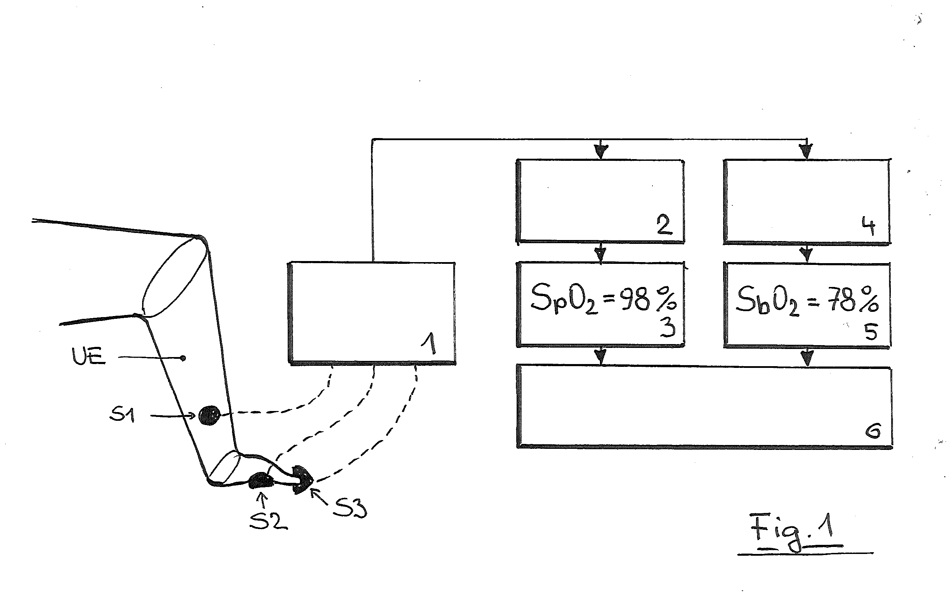

Figur 1 zeigt schematisch drei Ausführungsbeispiele der bevorzugten Sensorbefestigung (S1: reines Reflexionsmessprinzip; S2: kombiniertes Reflexions-Transmissionsmessprinzip; S3: reines Transmissionsmessprinzip) an der unteren Extremität (UE) des sitzenden Patienten und das Blockschaltbild der erfindungsgemäßen Messvorrichtung. Der Patient führt dabei eine standardisierte Beinübung (beispielsweise mit vorgegebener Frequenz, beispielsweise durch ein Metronom) im Sitzen vor. Im Elektronikblock (1) findet eine sensornahe Signalvorverarbeitung und Digitalisierung ohne jegliche Signalfilterung statt. Am Ausgang von (1) stehen zwei gleichzeitig bei $\lambda_1$ (bevorzugt 940 nm) und $\lambda_2$ (bevorzugt 660 nm) aufgenommene Perfusionssignale zur Verfügung, die kombinierte arterielle und venöse Blutvolumenschwankungen in der Extremität widerspiegeln. Sie werden weitergeleitet an zwei digitale Filtergruppen (2) und (4) mit unterschiedlichen spektralen Signaleigenschaften. Eine der Filtergruppen filtert und errechnet DC- und AC-Anteile des arteriellen Signalanteils, die andere DC- und AC-Signalanteile des venösen Signalanteils. Die Signalkomponenten am Ausgang der Filtergruppen (2) und (4) werden weiteren Elektronik- und Rechnergruppen (3) und (4) zugeführt und dort verschiedenen Algorithmen zur Berechnung der Sättigungswerte zugefügt. Die Einheit (3) ermittelt selektiv den arteriellen, herzschlagsynchronen Sättigungswert ($S_pO_2$, dieser liegt typischerweise bei 98 %), die Einheit (5) dann den venösen, bewegungskorrelierten Sättigungswert ($S_bO_2$, dieser liegt typischerweise bei 78%). In der Einheit wird abschließend die arterio-venöse Sättigungsdifferenz errechnet und angezeigt; sie beträgt in diesem Falle 20 %.

Typische arterielle Photoplethysmogramme, gleichzeitig bei zwei verschiedenen Messwellenlängen $\lambda_1$ und $\lambda_2$ bei ruhendem Bein gemessen, zeigt die Figur 2. Eingetragen dort sind ebenfalls die zugehörigen DC- und AC-Signalgrößen.

Die Sättigungswerte können in dem Fachmann bekannter Weise durch Bildung von Verhältnissen von AC- und DC-Anteilen bei den unterschiedlichen Wellenlängen bestimmt werden. Zunächst gilt für den sog. R-Wert:

$$R = f(SO_2) = \frac{(AC / DC)\lambda_2}{(AC / DC)\lambda_1}$$

wobei der Nenner das Verhältnis von AC- zu DC-Anteil bei der Messwellenlänge $\lambda_1$ und der Zähler das entsprechende Verhältnis bei der Messwellenlänge $\lambda_2$ angibt. Anhand der arteriellen AC/DC-Anteile kann ein arterieller R-Wert und anhand der venösen AC/DC-Anteile entsprechend ein venöser R-Wert bestimmt werden.

Mit Hilfe von in der Literatur beschriebenen analytischen Approximationen lässt sich aus dem R-Wert die Sauerstoffsättigung im Blut (venös oder arteriell) z. B. wie folgt errechnen:

$$SO_2 = (A - B \cdot R)\%$$

mit A = 110 und B = 25.
(Berechnungsvorschrift nach Rusch et al, Comput. Biol. Med. 26 (1996).

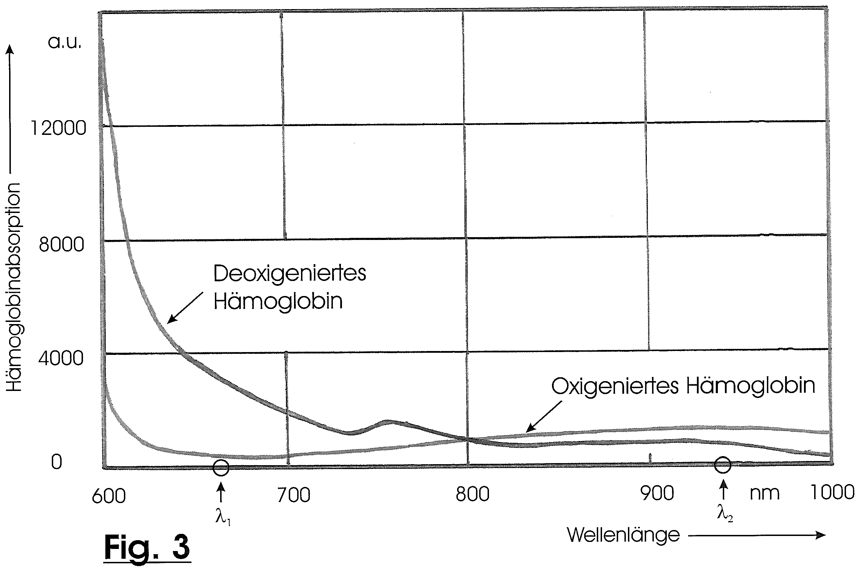

Figur 3 demonstriert das unterschiedliche molare Extinktionsverhalten vom oxigenierten und reduzierten Hämoglobin im Wellenlängenbereich vom 600 bis 1000 nm. Man erkennt daraus, dass sich die beiden Kurven bei $\lambda$ = 800 nm, dem sogenannten isosbestischem Punkt) kreuzen, hier ist die Lichtabsorption der beiden Blutderivate identisch.

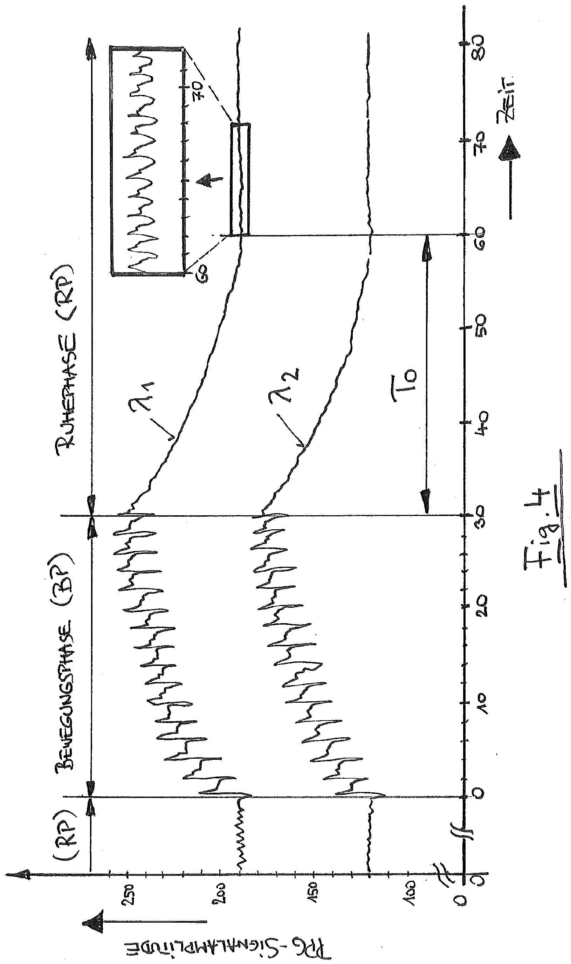

Typische ungefilterte Perfusionssignale (mit arteriellen und venösen Signalanteilen), gemessen an zwei verschiedenen Wellenlängen, können in schematischer Darstellung der Figur 4 entnommen werden. Gemessen wurde hier in drei aufeinander folgenden Phasen; einer initialen Ruhephase folgt eine 30 Sekunden lange Beinbewegungsphase und anschließend wieder eine Ruhephase. Die venöse Auffüllzeit beträgt in diesem Fall 30 Sekunden. Die Frequenz der beinübungkorrelierten Blutvolumenänderung beträgt 0,5 Hz, die Herzfrequenz 1 Hz. Diese liegen genügend weit auseinander, um elektronisch durch Filterung (vgl. die Filter (2) und (4) der Fig. 1) sicher getrennt zu können.

Figur 5 zeigt gemessene venöse Signalkomponenten. Neben dem photoplethysmographischen Gesamtsignal (a) sind der durch digitale Filterung daraus ermittelte venöse DC-Anteil (b) und der venöse AC-Anteil (c) dargestellt.

In der Figur 6 werden schließlich zwei bevorzugte Sensorapplikationspositionen am Bein / Fuß eines Probanden gezeigt. Durch die flexible Ausführung des Sensors ist eine Anpassung der Sensoroberfläche an die Beinform garantiert.

**Patentansprüche**

1. Verfahren zur nichtinvasiven Bestimmung der venösen Sauerstoffsättigung ($S_bO_2$), umfassend die folgenden Schritte:

   Beaufschlagen eines Messareals einer Extremität (UE), in welcher zumindest zeitweise durch passives und/oder aktives Bewegen der Extremität (UE) eine bewegungskorrelierte Blutvolumenänderung hervorgerufen wird, mit Strahlung mit mindestens zwei Messwelenlängen ($\lambda_1$, $\lambda_2$).
   Empfangen der reflektierten und/oder zurückgestreuten und/oder transmittierten Strahlung mit mindestens einem Detektor und
   Analysieren mindestens eines Detektorsignals, sodass ein DC- und ein AC-Anteil der venösen, bewegungskorrelierten Blutvolumenänderung in der Extremität (UE) bei jeder der verwendeten Messwellenlängen ($\lambda_1$, $\lambda_2$) selektiv ermittelt wird, Bestimmen der venösen Sauerstoffsättigung basierend auf dem ermittelten DC- und AC-Anteil.

2. Verfahren nach Anspruch 1, ferner umfassend das zumindest zeitweise passive und/oder aktive Bewegen der Extremität (UE).

3. Verfahren nach einem der Ansprüche 1 bis 2,
   **dadurch gekennzeichnet, dass** die dermalvenöse Sauerstoffsättigung bestimmt wird, insbesondere die Sauerstoffsättigung von oberen und/oder unteren Extremitäten (UE), insbesondere peripherer Beingebiete.

4. Verfahren nach einem der Ansprüche 1 bis 3,
   **dadurch gekennzeichnet, dass** das Analysieren von einer Auswerteeinheit durchgeführt wird, die detektornah angeordnet ist und keine spektralen Manipulationen an dem mindestens einen gemessenen (elektrischen) Detektorausgangssignal, insbesondere dem Perfusionssignal, stattfinden, sodass insbesondere hämodynamische informationsverluste reduziert, insbesondere ausgeschlossen, werden.

5. Verfahren nach einem der Ansprüche 1 bis 4,
   **dadurch gekennzeichnet, dass** das mindestens eine Detektorausgangssignal einer digitalen Filtereinheit (4) zugeführt wird, wo zumindest durch frequenzselektive Bewertung die DC- und AC-Anteile der venösen (Perfusions-)Signale errechnet werden, vorzugsweise für alle verwendeten Messwellenlängen ($\lambda_1$, $\lambda_2$) getrennt.

6. Verfahren nach Anspruch 5,
   wobei in einer weiteren digitalen Filtereinheit (2) die DC- und AC-Anteile der arteriellen (Perfusions-)Signale errechnet werden, vorzugsweise für alle verwendeten Messwellenlängen ($\lambda_1$, $\lambda_2$) getrennt, vorzugsweise beruhend auf einem Ausgangssignal des Detektors in einer Phase vor oder nach einer Phase, in der die passive und/oder aktive Bewegung der Extremität (UE) stattfindet.

7. Verfahren nach einem der Ansprüche 1 bis 6,
   **dadurch gekennzeichnet, dass** aus venösen und arteriellen DC- und AC-Wertepaaren sowohl die arterielle Sauerstoffsättigung ($S_bO_2$) als auch die venöse Sauerstoffsättigung ($S_bO_2$) bestimmt wird.

8. Verfahren nach Anspruch 7,
   **dadurch gekennzeichnet, dass** aus den Werten der arteriellen Sauerstoffsättigung ($S_pO_2$) und venösen Sauerstoffsättigung ($S_bO_2$) transkutan die Sauerstoffsättigungsdifferenz zwischen dem arteriellen und dem venösen Gefäßbett bestimmt wird.

9. Verfahren nach einem der Ansprüche 1 bis 8,
   **dadurch gekennzeichnet, dass** die Bewegung der Extremität (UE) eine aktive Beinübung durch den Patienten und/oder eine externe Beinübung durch den Untersucher (manuelle Blutvolumendrainage) und/oder eine passive Beinübung durch wiederholte Hoch- und Tieflagerung der Extremität (UE) ist.

10. Verfahren nach einem der Ansprüche: 1 bis 9, **dadurch gekennzeichnet, dass** die Außenseite einer Sensoroberfläche (weg vom Gewebe) eines Sensors, der den Detektor umfasst, aus lichtundurchlässigem Material ausgebildet ist und zwischen zwei äußeren flexiblen Sensorschichten des Sensors ebenfalls flexible leitende und geerdete Abschirmungsfolien eingearbeitet sind.

11. Verfahren nach einem der Ansprüche 1 bis 10, **dadurch gekennzeichnet, dass** die Frequenz der Bewegung der Extremität (UE) geringer als 1 Hz ist, vorzugsweise etwa 0,5 Hz.

12. Verfahren nach einem der Ansprüche 1 bis 11, **dadurch gekennzeichnet, dass** neben der Bestimmung der venösen Sauerstoffversorgung auch die venöse Auffüllzeit (To) bestimmt wird.

13. Verfahren nach Anspruch 1, **dadurch gekennzeichnet, dass**
die Strahlungsquelle(n) und der Detektor von einem Sensor (S1, S2, S3) umfasst sind, als Strahlungsquellen zwei selektive Lichtquellen verwendet werden und der Detektor ein Lichtdetektor ist,
das Analysieren von einer Steuer- und Auswerteelektronik durchgeführt wird, das Analysieren des mindestens einen Detektorausgangssignals Digitalisieren und Filtern umfasst,
die venöse Sauerstoffsättigung ($S_bO_2$) eine dermalvenöse Sauerstoffsättigung peripherer Beingebiete ist,
das Licht von dem Messareal und den darunterliegenden Schichten reflektiert respektive zurückgestreut wird,
ein Sensor (S1, S2, S3) in flacher und/oder biegsamer Bauweise ausgeführt ist, so dass seine Anpassung an das gewählte Gewebearealprofil gewährleistet ist und somit die Detektion der Strahlung sowohl im Reflexions- als auch Transmissionsmodus stattfinden kann, und
die Detektorausgangssignale derart digitalisiert und gefiltert werden, dass der DC- und AC-Anteil bei jeder der verwendeten Messwellenlängen ($\lambda_1$, $\lambda_2$) selektiv ermittelt werden kann, was die Bestimmung der venösen Sauerstoffsättigung ($S_bO_2$) im transilluminierten Gewebevolumen unter dem Sensor (S1, S2, S3) ermöglicht.

14. Vorrichtung oder System zur nichtinvasiven Bestimmung der venösen Sauerstoffsättigung ($S_bO_2$),
mit mindestens einer, vorzugsweise zwei Strahlungsquellen zur Erzeugung von Strahlung mit mindestens zwei Messwellenlängen ($\lambda_1$, $\lambda_2$), wobei die Strahlung ein Messareal einer Extremität (UE) beaufschlagt, mit mindestens einem Detektor, der die reflektierte und/oder zurückgestreute und/oder transmittierte Strahlung empfängt, und
mit einer mit dem Detektor verbundenen Auswerteelektronik zur Analyse mindestens eines Detektorausgangssignals, **dadurch gekennzeichnet, dass** die Auswerteelektronik zumindest eine ersten Filtereinheit aufweist und dazu eingerichtet ist, das mindestens eine Detektorausgangssignal derart zu analysieren, dass ein DC- und ein AC-Anteil der venösen, bewegungskorrelierten Blutvolumenänderung in der Extremität (UE) bei jeder der verwendeten Messwellenlängen ($\lambda_1$, $\lambda_2$) selektiv ermittelbar ist, was wiederum die Bestimmung der venösen Sauerstoffsättigung ($S_bO_2$) ermöglicht, wobei die bewegungskorrelierte Blutvolumenänderung durch passive und/oder aktive Bewegung der Extremität (UE) verursacht ist, wobei die erste Filtereinheit an eine Frequenz des venösen Signals syncnron mit der oder gesteuert durch die bewegung der Extremität angepasst ist und durch frequenzselektive Bewenung aen DC- und den AC-Anteil ermittelt.

15. Verwendung einer Vorrichtung oder eines Systems zur nichtinvasiven Bestimmung der venösen Sauerstoffsättigung ($S_b O_2$) einer Extremität (UE), in der zumindest zeitweise durch passive und/oder aktive Bewegung eine bewegungskorrelierte Blutvolumenänderung verursacht wird, wobei die Vorrichtung oder das System folgendes umfasst:
mindestens eine, vorzugsweise zwei Strahlungsquellen zur Erzeugung von Strahlung mit mindestens zwei Messwellenlängen ($\lambda_1$, $\lambda_2$), wobei die Strahlung ein Messareal der Extremität (UE) beaufschlagt, mindestens einen Detektor, der die reflektierte und/oder zurückgestreute und/oder transmittierte Strahlung empfängt, und eine mit dem Detektor verbundene Auswerteelektronik zur Analyse mindestens eines Detektorausgangssignals, wobei die Auswerteelektronik eingerichtet ist, das mindestens eine Detektorausgangssignal derart zu analysieren, dass ein DC- und ein AC-Anteil der venösen, bewegungskorrelierten Blutvolumenänderung in der Extremität (UE) bei jeder der verwendeten Messwellenlängen ($\lambda_1$, $\lambda_2$) selektiv ermittelt wird, und darauf basierend die Bestimmung der venösen Sauerstoffsättigung ($S_b O_2$) zu ermöglichen.

**Claims**

1. A method for non-invasive determination of venous oxygen saturation ($S_bO_2$) comprising the following steps:

   exposing a measurement area of an extremity (UE), in which at least temporarily a movement-correlated blood volume change is caused by passive and/or active movement of the extremity (UE), to radiation with at least two measurement wavelengths ($\lambda_1$, $\lambda_2$),
   receiving the reflected and/or backscattered and/or transmitted radiation with at least one detector and analysing at least one detector signal such that a DC and an AC component of the venous movement-correlated blood volume change in the extremity (UE) is selectively determined at each of the measurement wavelengths ($\lambda_1$, $\lambda_2$) used, determining the venous oxygen saturation based on the determined DC and AC component.

2. The method according to claim 1, further comprising at least temporarily passively and/or actively moving the extremity (UE).

3. The method according to one of claims 1 to 2,
   **characterised in that** the dermal venous oxygen saturation is determined, in particular the oxygen saturation of upper and/or lower extremities [UE), in particular peripheral leg areas.

4. The method according to one of claims 1 to 3,
   **characterised in that** the analysing is carried out by an evaluation unit arranged close to the detector and no spectral manipulations take place on the at least one measured (electrical) detector output signal, in particular the perfusion signal, so that in particular haemodynamic information losses are reduced, in particular excluded.

5. The method according to one of claims 1 to 4,
   **characterised in that** the at least one detector output signal is fed to a digital filter unit (4) where, at least by frequency-selective evaluation, the DC and AC components of the venous (perfusion) signals are calculated, preferably separately for all measurement wavelengths ($\lambda_1$, $\lambda_2$) used.

6. The method according to claim 5,
   wherein in a further digital filter unit (2), the DC and AC components of the arterial (perfusion) signals are calculated, preferably separately for all measurement wavelengths ($\lambda_1$, $\lambda_2$) used, preferably based on an output signal of the detector in a phase before or after a phase in which the passive and/or active movement of the extremity (UE) takes place.

7. The method according to one of claims 1 to 6,
   **characterized in that** from venous and arterial DC and AC value pairs, both the arterial oxygen saturation ($S_pO_2$) and the venous oxygen saturation ($S_bO_2$) are determined.

8. The method according to claim 7,
   **characterised in that** from the values of the arterial oxygen saturation ($S_pO_2$) and the venous oxygen saturation ($S_bO_2$) values, the oxygen saturation difference between the arterial and venous vascular beds is determined transcutaneously.

9. The method according to one of claims 1 to 8,
   **characterized in that** the movement of the extremity (UE) is an active leg exercise by the patient and/or an external leg exercise by the examiner (manual blood volume drainage) and/or a passive leg exercise by repeated raising and lowering of the extremity (UE).

10. The method according to one of claims 1 to 9,
    **characterised in that** the outside of a sensor surface (away from the tissue) of a sensor comprising the detector is formed of opaque material and likewise flexible conductive and grounded shielding foils are incorporated between two outer flexible sensor layers of the sensor.

11. The method according to one of claims 1 to 10,
    **characterized in that** the frequency of movement of the extremity (UE) is less than 1 Hz, preferably about 0.5 Hz.

12. The method according to one of claims 1 to 11,

**characterized in that**, in addition to determining the venous oxygen supply, the venous refilling time (To) is also determined.

13. The method according to claim 1, **characterized in that**
the radiation source(s) and the detector are comprised by a sensor (S1, S2, S3), two selective light sources are used as radiation sources and the detector is a light detector,
the analysing is carried out by control and evaluation electronics, the analysing of the at least one detector output signal comprises digitising and filtering, the venous oxygen saturation ($S_bO_2$) is a dermal venous oxygen saturation of peripheral leg areas,
the light is reflected and/or backscattered by the measurement area and the underlying layers,
a sensor (S1, S2, S3) is of flat and/or flexible construction so that its adaptation to the selected tissue area profile is ensured and thus the detection of the radiation can take place both in reflection and transmission mode, and
the detector output signals are digitised and filtered in such a way that the DC and AC components can be selectively determined at each of the measurement wavelengths ($\lambda_1$, $\lambda_2$) used, which enables the determination of the venous oxygen saturation ($S_bO_2$) in the transiltuminated tissue volume under the sensor (S1, S2, S3).

14. Device or system for non-invasive determination of venous oxygen saturation ($S_bO_2$),
with at least one, preferably two radiation sources for generating radiation with at least two measurement wavelengths ($\lambda_1$, $\lambda_2$), wherein the radiation is applied on a measurement area of an extremity (UE),
with at least one detector which receives the reflected and/or backscattered and/or transmitted radiation, and
with evaluation electronics connected to the detector for analysing at least one detector output signal, **characterised in that**
the evaluation electronics have at least one first filter unit and are configured to analyse the at least one detector output signal in such a way that a DC and an AC component of the venous movement-correlated blood volume change in the extremity (UE) is selectively determinable at each of the measurement wavelengths ($\lambda_1$, $\lambda_2$) used, which in turn enables the determination of the venous oxygen saturation ($S_bO_2$), wherein the movement-correlated blood volume change is caused by passive and/or active movement of the extremity (UE), wherein the first filter unit is adapted to a frequency of the venous signal synchronous with or controlled by the movement of the extremity and determines the DC and the AC component by frequency-selective evaluation.

15. Use of a device or system for non-invasive determination of venous oxygen saturation ($S_bO_2$) of an extremity (UE), in which a movement-correlated blood volume change is caused at least temporarily by passive and/or active movement, wherein the device or system comprises:

at least one, preferably two radiation sources for generating radiation with at least two measurement wavelengths ($\lambda_1$, $\lambda_2$), wherein a measurement area of the extremity (UE) is exposed to the radiation,
at least one detector which receives the reflected and/or backscattered and/or transmitted radiation, and
evaluation electronics connected to the detector for analysing at least one detector output signal, wherein the evaluation electronics are configured to analyse the at least one detector output signal in such a way that a DC and an AC component of the venous movement-correlated blood volume change in the extremity (UE) is selectively determined at each of the measurement wavelengths ($\lambda_1$, $\lambda_2$) used, and, based on this, to enable the determination of the venous oxygen saturation ($S_bO_2$).

**Revendications**

1. Procédé pour la détermination non invasive de la saturation veineuse en oxygène ($S_bO_2$) comprenant les étapes suivantes :

l'exposition d'une zone de mesure d'une extrémité (UE), dans laquelle un changement de volume sanguin corrélé au mouvement est provoqué au moins temporairement par un mouvement passif et/ou actif de l'extrémité (UE), à un rayonnement avec au moins deux longueurs d'onde de mesure ($\lambda_1$, $\lambda_2$),
la réception du rayonnement réfléchi et/ou rétrodiffusé et/ou transmis avec au moins un détecteur et
l'analyse d'au moins un signal de détecteur de sorte qu'une fraction DC et une fraction AC du changement de volume sanguin veineux corrélé au mouvement dans l'extrémité (UE) sont déterminées sélectivement à chacune des longueurs d'onde de mesure ($\lambda_1$, $\lambda_2$) utilisées,
la détermination de la saturation veineuse en oxygène sur la base de la fraction DC ét AC déterminée.

**2.** Procédé selon la revendication 1, comprenant en outre le mouvement au moins temporairement de l'extrémité (UE) de manière passive et/ou active.

**3.** Procédé selon l'une des revendications 1 à 2,
**caractérisé en ce que** la saturation en oxygène des veines dermiques est déterminée, en particulier la saturation en oxygène des extrémités supérieures et/ou inférieures (UE), en particulier des zones périphériques des jambes.

**4.** Procédé selon l'une des revendications 1 à 3,
**caractérisé en ce que** l'analyse est effectuée par une unité d'évaluation qui est disposée à proximité du détecteur et qu'aucune manipulation spectrale n'a lieu sur l'au moins un signal (électrique) mesuré de sortie du détecteur, en particulier le signal de perfusion, de sorte que les pertes d'informations, en particulier hémodynamiques, sont réduites, en particulier exclus.

**5.** Procédé selon l'une des revendications 1 à 4,
**caractérisé en ce que** l'au moins un signal de sortie du détecteur est envoyé à une unité de filtrage numérique (4), où, au moins par une évaluation sélective en fréquence, les fractions DC et AC des signaux veineux (de perfusion) sont calculées, de préférence séparément pour toutes les longueurs d'onde de mesure ($\lambda_1$, $\lambda_2$) utilisées,

**6.** Procédé selon la revendication 5,
dans lequel, dans une autre unité de filtrage numérique (2), les fractions DC et AC des signaux artériels (de perfusion) sont calculées, de préférence séparément pour toutes les longueurs d'onde de mesure ($\lambda_1$, $\lambda_2$) utilisées, de préférence sur la base d'un signal de sortie du détecteur dans une phase avant ou après une phase dans laquelle le mouvement passif et/ou actif de l'extrémité (UE) a lieu.

**7.** Procédé selon l'une des revendications 1 à 6,
**caractérisé en ce que**, à partir des paires veineuses et artérielles de valeurs DC et AC, la saturation artérielle en oxygène ($S_pO_2$) et la saturation veineuse en oxygène ($S_bO_2$) sont déterminées toutes deux.

**8.** Procédé selon la revendication 7,
**caractérisé en ce que** la différence de saturation en oxygène entre les lits vasculaires artériels et veineux est déterminée de manière transcutanée à partir des valeurs de la saturation artérielle en oxygène ($S_pO_2$) et la saturation veineuse en oxygène ($S_bO_2$).

**9.** Procédé selon l'une des revendications 1 à 8,
**caractérisé en ce que** le mouvement de l'extrémité (UE) est un exercice de jambe actif par le patient et/ou un exercice de jambe externe par l'examinateur (drainage manuel du volume sanguin) et/ou un exercice de jambe passif par élévation etabaissement répétés de l'extrémité (UE).

**10.** Procédé selon l'une des revendications 1 à 9,
**caractérisé en ce que** le côté extérieur d'une surface de capteur (en l'éloignant du tissu) d'un capteur comprenant le détecteur est formé d'un matériau opaque et des feuilles de blindage également flexibles, conductrices et mises à la terre sont incorporées entre deux couches de capteur extérieures flexibles du capteur.

**11.** Procédé selon l'une des revendications 1 à 10,
**caractérisé en ce que** la fréquence de mouvement de l'extrémité (UE) est inférieure à 1 Hz, de préférence environ 0,5 Hz.

**12.** Procédé selon l'une des revendications 1 à 11,
**caractérisé en ce que**, en plus de la détermination de l'approvisionnement veineuse d'oxygène, le temps de remplissage veineux (To) est déterminé également.

**13.** Procédé selon la revendication 1, **caractérisé en ce que**
la (ou les) source(s) de rayonnement et le détecteur sont compris d'un capteur (S1, S2, S3), deux sources de lumière sélectives sont utilisées comme sources de rayonnement et le détecteur est un détecteur de lumière,
l'analyse est effectuée par une électronique de commande et d'évaluation, l'analyse de l'au moins un signal de sortie du détecteur comprend la numérisation et le filtrage,
la saturation veineuse en oxygène ($S_bO_2$) est une saturation en oxygène des veines dermiques des zones périphériques des jambes,

la lumière est réfléchie ou rétrodiffusée, respectivement, par la zone de mesure et les couches sous-jacentes, un capteur (S1, S2, S3) est de construction plate et/ou flexible, de sorte que son adaptation au profil de la zone tissulaire sélectionnée est assurée et qu'ainsi la détection du rayonnement peut avoir lieu aussi bien en mode réflexion qu'en mode transmission, et

les signaux de sortie du détecteur sont numérisés et filtrés de telle sorte que les fractions DC et AC peuvent être déterminées sélectivement à chacune des longueurs d'onde de mesure ($\lambda_1$, $\lambda_2$) utilisées, ce qui permet de déterminer la saturation veineuse en oxygène ($S_bO_2$) dans le volume tissulaire transilluminé sous le capteur (S1, S2, S3).

**14.** Dispositif ou système pour la détermination non invasive de la saturation veineuse en oxygène ($S_bO_2$), avec au moins un, de préférence deux sources de rayonnement pour générer un rayonnement avec au moins deux longueurs d'onde de mesure ($\lambda_1$, $\lambda_2$), le rayonnement étant appliqué sur une zone de mesure d'une extrémité (UE), avec au moins un détecteur qui reçoit le rayonnement réfléchi et/ou rétrodiffusé et/ou transmis, et avec une électronique d'évaluation reliée au détecteur pour analyser au moins un signal de sortie du détecteur, **caractérisé en ce que**

l'électronique d'évaluation présente au moins une première unité de filtrage et est conçue pour analyser l'au moins un signal de sortie du détecteur de telle sorte qu'une fraction DC et Une fraction AC du changement de volume sanguin veineux corrélé au mouvement dans l'extrémité (UE) puissent être déterminées sélectivement à chacune des longueurs d'onde de mesure ($\lambda_1$, $\lambda_2$) utilisées, ce qui permet à son tour de déterminer la saturation veineuse en oxygène ($S_bO_2$), le changement de volume sanguin corrélé au mouvement étant provoqué par un mouvement passif et/ou actif de l'extrémité (UE), la première unité de filtrage étant adaptée à une fréquence du signal veineux synchrone avec ou contrôlée par le mouvement de l'extrémité et déterminant les fractions DC et AC par une évaluation sélective en fréquence.

**15.** Utilisation d'un dispositif ou d'un système pour la détermination non invasive de la saturation veineuse en oxygène ($S_bO_2$) d'une extrémité (UE), dans laquelle un changement de volume sanguin corrélé au mouvement est provoqué au moins temporairement par un mouvement passif et/ou actif, le dispositif ou le système comprenant ; au moins une, de préférence deux sources de rayonnement pour générer un rayonnement avec au moins deux longueurs d'onde de mesure ($\lambda_1$, $\lambda_2$), le rayonnement étant appliqué sur une zone de mesure de l'extrémité (UE), au moins un détecteur qui reçoit le rayonnement réfléchi et/ou rétrodiffusé et/ou transmis, et une électronique d'évaluation reliée au détecteur pour analyser au moins un signal de sortie du détecteur, l'électronique d'évaluation étant conçue pour analyser l'au moins un signal de sortie du détecteur de telle sorte qu'une fraction DC et une fraction AC du changement de volume sanguin veineux corrélée au mouvement dans l'extrémité (UE) soient déterminées sélectivement à chacune des longueurs d'onde de mesure ($\lambda_1$, $\lambda_2$) utilisées, et pour permettre, sur cette base, la détermination de la saturation veineuse en oxygène ($S_bO_2$).

$S_pO_2 = 98\%$

$S_bO_2 = 78\%$

UE

S1

S2

S3

Fig.1

EP 2 609 854 B1

Fig. 2

**Fig. 3**

EP 2 609 854 B1

Fig. 4

EP 2 609 854 B1

Fig.5

**Fig. 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3100610

- US 6334065 B1

**In der Beschreibung aufgeführte Nicht-Patentliteratur**

- **RUSCH et al.** *Comput. Biol. Med.,* 1996, vol. 26